# EUROPÄISCHE PATENTSCHRIFT

(11) **EP 0 493 363 B1**
(45) Veröffentlichungstag und Bekanntmachung des Hinweises auf die Patenterteilung: **02.06.1999**
(21) Anmeldenummer: 92102656.3
(22) Anmeldetag: 20.03.1987
(51) Int. Cl.: A61F 2/46

(54) **Vorrichtung und Verfahren zum Mischen und Umfüllen eines viskösen Stoffgemisches**
Device and method for mixing and transferring a viscous mixture of substances
Dispositif et procédé pour mélanger et transvaser un mélange de matières visqueuses

(30) Priorität: 21.03.1986 DE 3609672
(43) Veröffentlichungstag der Anmeldung: 01.07.1992
(62) Teilanmeldung aus: 87902050.1
(73) Patentinhaber: DRAENERT, Klaus, Dr.med., D-81545 München (DE)
(72) Erfinder: DRAENERT, Klaus, Dr.med., D-81545 München (DE)

(56) Entgegenhaltungen:
- EP-A- 0 170 120
- DE-A- 3 347 843
- US-A- 3 779 371

## Beschreibung

Die Erfindung betrifft eine Vorrichtung und ein Verfahren zum Mischen und Umfüllen eines aus mindestens zwei Bestandteilen bestehenden viskösen Stoffgemisches. Insbesondere betrifft die Erfindung eine Vorrichtung und ein Verfahren zum Mischen und Umfüllen von Knochenzement von einem Mischbecher in einen Applikationsbehälter.

Unter Mischen versteht man allgemein das Einbringen von Teilen eines Stoffes zwischen die Teile anderer Stoffe. Ziel ist dabei eine möglichst homogene Verteilung der verschiedenen Bestandteile des Stoffgemisches, um beispielsweise eine chemische Reaktion zwischen den Bestandteilen einzuleiten oder zu fördern, z. B. die nachfolgende Polymerisation eines Zwei- oder Mehrkomponentengemisches.

Es sind verschiedene Arten von Mischverfahren und -vorrichtungen bekannt. Das Mischen erfolgt dabei beispielsweise durch Rühren, Mengen, Walzen, Kneten, Emulgieren, Suspendieren, Lösen oder durch Einwirkung von Ultraschall.

Die bekannten Mischverfahren und -vorrichtungen weisen jedoch den Nachteil auf, daß Verunreinigungen, beispielsweise Luft, in das Mischsystem eingetragen werden können und daß die bereits zu Beginn des Mischvorganges in den zu mischenden Stoffen enthaltenen und die während des Mischvorganges, beispielsweise durch chemische Reaktionen entstehenden Gaseinschlüsse nicht aus dem Mischsystem entfernt werden können. Ferner besteht ein Nachteil häufig darin, daß der Mischbehälter nicht mit dem Behälter identisch ist, in dem die weitere Verarbeitung des durchmischten Stoffgemisches erfolgen soll. Es ist dann ein Transport des Stoffgemisches erforderlich, der insbesondere bei im Stoffgemisch ablaufenden Reaktionen, beispielsweise einer Polymerisation, Probleme bereitet.

Aus der US-A-3779371 ist eine Vorrichtung bekannt, die eine Pulverkomponente mit einer Flüssigkeit mischt (Spalte 2, 14 - 18). Die Flüssigkeit wird über einen Druckkolben über ventilartige Öffnungen und Schlitze überfuhrt, und das Mischen erfolgt durch Schütteln der gesamten Anordnung. Dies läßt jedoch keine homogenen Mischungen erreichen, da bekannt ist, daß ausschließlich leicht lösliche Substanzen durch Schütteln gemischt werden können, was bei Zweikomponentenmaterialien, die Homogenität voraussetzen, auszuschließen ist.

In der DE-A-3347843 wird ein Zweikomponentenmischbehälter beschrieben, der über eine Vorrichtung den Flüssigkeitsbehälter öffnen kann und durch Auf- und Abbewegen einer Mischerscheibe im selben Behälter anmischt. Auch bei diesem Verfahren kann nicht ausreichend gemischt werden, und es kann nur da Anwendung finden, wo Homogenität nicht gefragt ist.

Spezielle Probleme treten beim Verarbeiten und Mischen von Knochenzement und beim Einfüllen oder Umfüllen in den Behälter auf, aus dem der Knochenzement appliziert wird.

Als Knochenzement werden meist kalt polymerisierende Zweikomponenten-Kunststoffe verwendet, durch die die Komponenten künstlicher Gelenke im knöchernen Bett verankert werden. Der Knochenzement härtet unmittelbar nach der Applikation aus und erreicht durch seine plastischen Eigenschaften eine Verblockung der Prothesenkomponente im knöchernen Lager. Seit vielen Jahren werden als Knochenzemente Polymethylmethacrylate (PMMA) verwendet. Diese bestehen aus einem pulverförmigen Perlpolymerisat, welches in flüssigem Monomer angelöst und schließlich durch Polymerisation des Monomers in dieses eingebettet wird. In der Mischphase umfließt das Monomer das etwa kugelförmige Polymerpulver. Dabei gibt es zunächst eine Kugelaufschwemmung, in der mehr oder weniger viele Luftblasen eingeschlossen sind. Die Polymerisation läuft exotherm ab. Neben den eingeschlossenen Luftblasen kommt es regelmäßig beim Umfließen der Polymerkugeln durch das Monomer zu sogenannten "Windschattenphänomenen", also einer unzureichenden Benetzung der Polymerkugeln, und während der exothermen Polymerisation auch zum Verdampfen der monomeren Flüssigkeit, so daß letztlich der ausgehärtete Knochenzement von Blasen unterschiedlicher Ethiologie und Genese durchsetzt ist.

In der Regel wird das Polymerpulver dem Monomer beigegeben und mit einem Spatel in einer Schale vermischt. In der an die Mischphase anschließenden Verarbeitungsphase wird der Knochenzement meist von Hand, teilweise auch mit einer Spritze, in das knöcherne Lager eingebracht, beispielsweise in die Femurmarkhöhle oder in das knöcherne Acetabulum, die für die Verankerung der zementierten Prothesenkomponenten vorbereitet wurden. Eine derartige Spritze ist beispielsweise in dar DE-A-28 01 706 oder in der EP-A1-170 120 beschrieben. Durch Verwendung einer Knochenzementspritze konnten im Hinblick auf die Zementverankerung im Knochen wesentlich bessere Ergebnisse als mit der herkömmlichen Fingerstopfmethode erzielt werden.

Es gibt bisher kaum Arbeiten, die sich mit der Misch- oder Quellphase des Knochenzementes und dem artefaktfreien Einfüllen oder Umfüllen in das Spritzensystem befassen.

Die weitere Verarbeitung des in der vorstehend erläuterten Weise in der Mischschale angerührten Knochenzementes hängt von dessen Viskosität ab. Sehr niedrigvisköse Knochenzemente können aus der Schale in die Kartusche der Knochenzementspritze gegossen werden; hierbei tritt jedoch das Problem auf, daß der einfließende Knochenzementstrahl sehr leicht, z.B. durch elektrostatische Aufladung abgelenkt wird, so daß fast immer die Wand der Kartusche und die Einfüllöffnung mit Knochenzement verschmiert werden. Hochvisköse Zemente lassen sich überhaupt nicht gießen. Sie müssen von Hand herausgenommen und geknetet werden, damit die gröbsten Lufteinschlüsse herausgedrückt werden. Anschließend wird der Knochenzement zu einer wurstförmigen Masse gerollt, die dann in die Kartusche eingeführt werden kann. Die Handverarbeitung des Zementes erfordert nicht nur ein Warten, bis der Knochenzement nicht mehr an den Operationshandschuhen kleben bleibt, sondern läßt die Zementmischung auch in der wertvollsten Zeit der Quellphase und der sich daran anschließenden Vorpolymerisationsphase unbearbeitet.

Die bisher vorgeschlagenen Versuche, das Problem des Zementmischens in einem sogenannten "geschlossenen System" in den Griff zu bekommen, führten zu keinen besseren Mischungen als die von Hand ausgeführten Ansätze.

Der Erfindung liegt somit die Aufgabe zugrunde, ein Verfahren und eine Vorrichtung bereitzustellen, mit denen ein aus mindestens zwei Bestandteilen bestehendes visköses Stoffgemisch rasch und blasenfrei durchmischt und in den Behälter eingeführt werden kann, in dem die weitere Verarbeitung des Stoffgemisches stattfinden soll.

Eine weitere Aufgabe der Erfindung besteht darin, eine Vorrichtung und ein Verfahren zum Mischen und Umfüllen von Knochenzement in einen Applikationsbehälter bereitzustellen, mit denen der aus mehreren Komponenten bestehende Knochenzement rasch, blasenfrei und ohne Berührung durch den Operateur durchmischt und in den Applikationsbehälter eingefüllt werden kann, aus dem heraus er in das knöcherne Lager appliziert werden soll.

Diese Aufgaben werden durch die Merkmale der Patentansprüche gelöst.

Die Erfindung geht dabei von dem Grundgedanken aus, die zu mischenden Stoffe , vorzugsweise ein aushärtbares Zweikomponentensystem, also beispielsweise das Gemisch aus Polymerpulver und Monomer, von einem ersten Behälter durch eine Öffnung oder Verjüngung in einen zweiten Behälter hineinzudrücken. Zur Verbesserung der Durchmischung wird das Stoffgemisch anschließend wieder vom zweiten Behälter durch die Öffnung in den ersten Behälter zurückgedrückt. Dieser Vorgang kann mehrfach wiederholt werden und läßt sich als "Extrusionsmischen" bezeichnen.

Vorzugsweise ist der erste Behälter oder Mischbehälter becherförmig ausgebildet bzw. zylindrisch mit einem geschlossenen und einem offenen Ende. Der zweite Behälter, der gleichzeitig als Applikationsbehälter dient, in dem das Stoffgemisch nach der Durchmischung zur Weiterverarbeitung verbleibt, ist vorzugsweise ebenfalls zylindrisch ausgebildet mit einem offenen Ende. Das andere Ende des zweiten Behälters ist vorzugsweise durch eine Kappe oder einen Stempel verschließbar. Der zweite Behälter trägt an seinem Außenumfang, vorzugsweise an seinem offenen Ende, eine Abdichteinrichtung, die vorzugsweise aus mehreren flexiblen Scheiben oder Lamellen besteht und mit dem zweiten Behälter koppelbar ist.

Zur Mischung und Umfüllung des Stoffgemisches wird der zweite zylindrische Behälter oder Hohlkörper mit seinem offenen Ende voran in Axialrichtung in den ersten Behälter hineingedrückt, in dem sich die zu mischenden oder bereits teilweise gemischten Stoffe befinden. Durch diese Relativbewegung der beiden Behälter wird das Stoffgemisch durch die Öffnung in den zweiten Behälter hineingedrückt, da die Abdichteinrichtung den Zwischenraum zwischen der Innenwand des ersten Behälters und der Außenwand des zweiten Behälters derart abschließt, daß lediglich Gas, aber nicht die zu mischenden Stoffe passieren können.

Bei der Anwendung des vorstehenden Prinzips auf das Mischen und Umfüllen von Knochenzement in einen Applikationsbehälter bzw. eine Kartusche wird zunächst das Monomer in einem Mischbecher vorgelegt und danach das Polymerpulver in den Ansatz gebracht und mit einem Spatel aus Metall, Kunststoff oder Holz untermischt und verrührt. Anstatt nun wie bei herkömmlichen Verfahren den gesamten Zementbrei mit dem Spatel herauszunehmen oder bei niedrigviskösen Zementen herauszugießen, wird erfindungsgemäß von der Öffnung des Mischbechers her der als Kartusche ausgebildete Applikationsbehälter mit der aufgesetzten Abdichteinrichtung eingesetzt. Wenn die Abdichteinrichtung als zylinderförmiger Körper mit zentraler, rohrförmiger Öffnung und mehreren scheibenförmigen Lamellen ausgebildet ist, kann die Abdichteinrichtung unter Einsetzen von Adaptern, vorzugsweise kleinen Ringen, auf alle handelsüblichen Kartuschen aufgesetzt und zusammen mit diesen verwendet werden. Vorzugsweise wird der in der EP-A1-170 120 erläuterte zylinderförmige Behälter als Applikationsbehälter bzw. Kartusche verwendet. Durch Eindrücken der Kartusche mit der damit gekoppelten Abdichteinrichtung wird der durch den Spatel vorgemischte Zement durch die zentrale Öffnung der Abdichteinrichtung in die Kartusche hineingedrückt. Die Öffnung kann dabei entweder den gleichen oder kleineren Durchmesser als die Kartusche aufweisen. Durch die Extrusionswirkung findet eine weitere Durchmischung des Knochenzementes statt und der Knochenzement gelangt ohne Berührung durch den Operateur vom Mischbecher in die Kartusche, wobei durch den Fließvorgang zusätzlich größere Luftblasen herausgedrückt werden.

Zum Vormischen des Zements wird anstelle herkömmlicher Spatel vorzugsweise ein Rundstab verwendet, der vorteilhafterweise mit Teflon beschichtet ist. Der Rundstab hat den Vorteil, daß er das Gemisch nicht zerreißt, sondern dessen Durchmischung durch eine laminare Strömung der Schichten des Gemisches fördert. Außerdem bleibt am Teflonrundstab beim Rühren und Herausziehen praktisch kein Zement haften.

Besonders vorteilhaft zur Vermeidung von Lufteinschlüssen ist es, das Mischen der Bestandteile, beispielsweise der Komponenten des Knochenzementes, unter Vakuum durchzuführen. Hierzu weist der Mischbehälter vorzugsweise einen plangeschliffenen oberen Rand auf, auf den ein Deckel aufgesetzt wird. Die Abdichtung zwischen dem Mischbehälter und dem Deckel kann durch einen Dichtring mit Vakuumfett oder eine Silikonbeschichtung erfolgen. Der Deckel kann auch durch eine rasch lösbare Flanschverbindung mit dem Mischbehälter vakuumdicht verbunden werden. Der Deckel weist einen Anschluß für eine Schlauchzuführung auf, an die eine Vakuumpumpe angeschlossen wird.

Vorzugsweise weist der Deckel einen festen Rand zur Auflage auf dem Rand des Mischbehälters und eine Durchführung für den zum Rühren verwendeten Rundstab auf. Die abgedichtete Durchführung für den Rundstab ist vorzugsweise innerhalb eines aus flexiblem Material bestehenden Innenteils des Deckels angeordnet, der mit dem festen Rand vakuumdicht, vorzugsweise einstückig verbunden ist. Das Innenteil des Deckels kann zeltförmig ausgebildet sein und an seinem oberen Ende die als eine ringförmige Führung ausgebildete Durchführung, vorzugsweise aus Gummi oder Silikon aufweisen, durch die der Rundstab geführt wird und die den Rundstab vakuumdicht festhält. Durch den flexiblen Innenteil des Deckels ist der Rundstab in Radialrichtung im Mischbehälter beweglich und kann an der Innenwand des Mischbehälters entlang geführt werden, so daß kein Teil der zu mischenden Bestandteile, beispielsweise kein Pulver des Knochenzements, unberührt an der Wand liegen bleibt. Dies ist für eine gute Durchmischung außerordentlich wichtig.

Durch die Mischung unter Vakuum können die Blasen im Zement noch weiter reduziert und das Gemisch praktisch blasenfrei gerührt werden.

Bei Versuchen hat sich herausgestellt, daß hohe Mischbecher zu einer sehr viel schnelleren und homogeneren Mischung der Zementmasse führen als weite, flache und an den Ecken totrandige Mischschalen. Vorzugsweise ist der Boden des Mischbechers innen sphärisch oder konkav ausgebildet. Beim Hineindrücken der Kartusche paßt sich die vorderste, flexible Lamelle der Abdichteinrichtung dem Innenboden des Mischbechers abdruckmäßig derart an, daß keine Reste im Mischbecher zurückbleiben. Besonders vorteilhaft ist es hierbei, wenn der Innenboden des Mischbechers ebenfalls flexibel ist, so daß eine vollständige Anpassung der Form gewährleistet ist.

Toträume während des Fließvorganges am oberen Rand der Zementmasse können dadurch verhindert werden, daß die vorderste Lamelle der Abdichteinrichtung ebenfalls etwas gekrümmt bzw. konkav ist, so daß die Zementmasse vom Außenrand des Mischbechers radial nach innen gedrückt wird.

Wie vorstehend erläutert, können während des Hineindrückens der Kartusche mit der Abdichteinrichtung Luft und die während der Polymerisation frei werdenden Gase an den Lamellen der Abdichteinrichtung vorbei entweichen. Der Körper der Abdichteinrichtung kann auch mindestens ein zusätzliches Ventil aufweisen, durch das die Gase ausweichen können.

Ferner kann auch in der Mischphase durch Anwendung von Druck und Verschließen der zentralen Öffnung oder des Kartuschenendes bei gleichzeitiger mechanischer Komprimierung des Zementes ein Vakuum angelegt und Luft aus dem Mischbecher abgesaugt werden, so daß auch kleine Luftblasen bereits während des Mischvorganges zum größten Teil aus dem Zement entfernt werden können.

Beim Einfüllen des Zementes in die Kartusche ist es wichtig, daß diese an ihrem vom Mischbecher abgewandten Ende nicht verschlossen ist oder, falls sie eine Kappe aufweist, die Kappe nur locker aufgesetzt ist, damit die vor dem Zement herausgedrückte Luft aus der Kartusche entweichen kann.

Bei dem vorstehend erläuterten System kann als zweiter Behälter unmittelbar eine Kartusche verwendet werden, wie sie in einer Knochenzementspritze gemäß der EP-A1-170 120 oder in ähnlichen Spritzen verwendet wird. Bei diesem Sysem muß der Zement bis zur Applikation überhaupt nicht mehr mit den Händen und Operationshandschuhen des Operateurs oder der Operationsschwester in Berührung kommen, wodurch große Vorteile erzielt werden. Zum einen ist es bekannt, daß das Monomer die Gummihandschuhe des Operationspersonals leicht durchdringen kann, und es wurden in letzter Zeit immer mehr Allergien gegen den Kunststoff bekannt. Zum anderen können durch das berührungsfreie Vorgehen auch die Fehleinschlüsse im Zement erheblich verringert werden und der Zement gelangt rascher und früher zur Vorkomprimierung in die Knochenzementpistole, weil das Abbinden des Zementes nicht abgewartet werden muß.

Eine noch bessere Durchmischung des Zementes läßt sich durch einen im zweiten Behälter beweglichen Stempel erzielen. Der Stempel oder Kolben schließt den zweiten Behälter innen zementdicht, aber gasdurchlässig ab und kann mittels einer Handhabungsvorrichtung axial in den zweiten Behälter hineingedrückt und aus ihm herausgezogen werden.

Mit diesem System läßt sich das Stoffgemisch, beispielsweise der Knochenzement, mehrfach durch die zentrale Öffnung zwischen dem ersten und zweiten Behälter hin- und herbewegen. Aufgrund der Strömung und der Extrusionswirkung erfolgt eine ausgezeichnete Durchmischung der Bestandteile. Nachdem das Stoffgemisch durch Hineindrücken des zweiten Behälters in den ersten Behälter durchmischt und in den ersten Behälter umgefüllt ist, wird der Stempel mittels der Handhabungsvorrichtung nach unten gestoßen, während gleichzeitig der erste Behälter festgehalten wird. Dadurch wird das Gemisch vom Stempel aus dem zweiten Behälter wieder in den ersten Behälter ausgestoßen, wobei sich der zweite Behälter entgegengesetzt zum Stempel und relativ zum ersten Behälter nach oben bewegt, da das Stoffgemisch Druck auf die vorderste Lamelle der Abdichteinrichtung ausübt und die mit dem zweiten Behälter gekoppelte Abdichteinrichtung nach oben drückt. Anschließend wird der zweite Behälter wieder in den ersten Behälter hineingestoßen, wobei das Stoffgemisch in den zweiten Behälter zurückströmt und den Stempel zurückdrückt. Dieser Vorgang kann mehrfach wiederholt werden, bis eine innige Durchmischung stattgefunden hat.

Bei dieser Ausführungsform ist es auch möglich, auf das vorhergehende Ansetzen und Rühren der Bestandteile des Stoffgemisches zu verzichten. Dies wird nachstehend beispielsweise anhand der Verwendung beim Mischen und Umfüllen von Knochenzement erläutert.

Das Polymerpulver wird zunächst in den zweiten Behälter oder Applikationsbehälter eingebracht, dessen untere Öffnung zunächst mit einer Membran verschlossen ist. Das andere Ende des Applikationsbehälters wird mit dem Stempel geschlossen, und der Applikationsbehälter wird bis zum Boden des als Mischbehälter dienenden ersten Behälters gedrückt.

Das Monomer befindet sich in einem dritten Behälter, der eine Spitze oder Nadel aufweist. Die Spitze kann zunächst geschlossen sein und wird erst kurz vor der Anwendung geöffnet. Die Spitze des Monomerbehälters wird durch einen am Boden des Mischbechers befindlichen wiederverschließbaren Stopfen gedrückt, und durch Zurückziehen des Applikationsbehälters wird aufgrund des entstehenden Unterdruckes das Monomer in den Mischbecher hineingesaugt. Danach wird der Monomerbehälter wieder abgezogen, so daß sich der Stopfen am Boden des Mischbechers wieder schließt. Nun löst das Monomer die aus einem geeigneten Material ausgebildete Membran auf, die bisher den Applikationsbehälter verschlossen hält, und das Polymerpulver wird befreit und kann durch den Stempel in das Monomer im Mischbecher hineingedrückt werden. Danach können das Monomer und das Polymerpulver durch mehrmaliges Hin- und Herbewegen von Stempel und Applikationsbehälter, wie vorstehend erläutert, innig durchmischt werden. Eingeschlossene Luft kann durch ein Ventil in der Abdichteinrichtung austreten.

Die vorstehend erläuterte Ausführungsform kann als "geschlossenes System" bezeichnet werden, da die Bestandteile des Stoffgemisches, wie z. B. des Knochenzementes, abgeschlossen und getrennt voneinander aufbewahrt werden können und während der gesamten Verarbeitung und Mischung bis zum Einfüllen in den Applikationsbehälter nicht mit dem Operateur in Berührung kommen und auch keinen Verarbeitungsschritten, wie z.B. Rühren mit einem Spatel unterzogen werden, bei denen Lufteinschlüsse erzeugt werden.

Vorzugsweise bestehen der erste Behälter (Mischbecher) und der zweite Behälter (Applikationsbehälter) aus demselben Material. Es kann ein Kunststoff, vorzugsweise ein thermoplastischer Kunststoff, wie ein Polyolefin verwendet werden. Besonders bevorzugt ist die Verwendung von Poly(4-methyl-1-penten) oder TPX®. Es kann auch Polycarbonat verwendet werden. Der dritte Behälter (Monomerbehälter) besteht aus einem Material das vom Monomer nicht angelöst wird, vorzugsweise aus Teflon.

Der Vakuumdeckel für den Mischbehälter besteht vorzugsweise ebenfalls aus Teflon oder dem für den Mischbehälter und den Applikationsbehälter verwendeten Material, beispielsweise Poly(4-methyl-1-penten).

Die Erfindung wird nachstehend anhand von Ausführungsbeispielen, die das Mischen und Umfüllen von Knochenzement in einen Applikationsbehälter betreffen, und anhand der Zeichnung näher erläutert. Es zeigen:
Figuren 1 bis 4 im Teilschnitt eine Ausführungsform einer erfindungsgemässen Vorrichtung mit Stempel in verschiedenen Stellungen während der Durchführung des erfindungsgemässen Verfahrens,
Fig.5 eine Handhabungsvorrichtung für die Vorrichtung gemäss den Figuren 1 bis 4 und
Fig 6 eine weitere Ausführungsform der erfindungsgemässen Vorrichtung in der Stellung gemäss Fig 1 mit zusätzlicher Vakuumleitung.

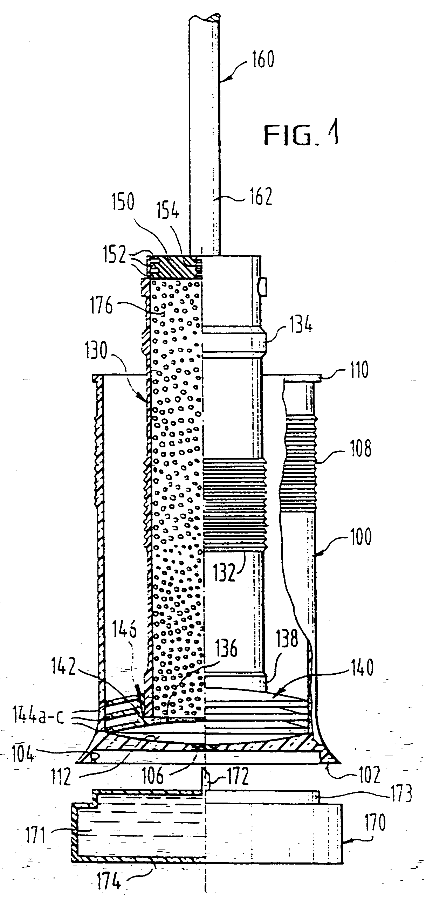

In Fig. 1 ist eine als 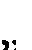 geschlossenes System" verwendbare Ausführungsform der Erfindung dargestellt.

Der kreisförmige Mischbecher 100 weist an seinem Boden eine Standfläche 102 mit einer kreisförmigen Ausnehmung 104 auf. Im Boden ist zentral ein Gummistopfen 106 vorgesehen. Seitlich am Umfang weist der Mischbecher 100 eine geriffelte Griffläche 108 und oben einen als Verstärkung dienenden Rand 110 auf Der innere Boden 112 des Mischbechers 100 ist konkav ausgebildet.

Der Applikationsbehälter 130 weist ebenfalls eine geriffelte Griffläche 132 sowie eine Rippe 134 auf Das untere Ende des Applikationsbehälters 130 ist mittels einer Membran 136 verschlossen., die einstückig mit dem Applikationsbehälter 130 oder mit eine mittels eines Adapters 138 mit dem Applikationsbehälter verbundenen Abdichteinrichtung 140 ist.

Die Abdichteinrichtung 140 ist aus Teflon hergestellt und weist einen zylindrischen Körper 142 und drei scheibenförmige, flexible Lamellen 144 a - c auf. Die Lamellen 144 schliessen gegen die Innenwand des Mischbechers 100 gasdurchlässig, aber flüssigkeits- und insbesondere zementdicht ab. Die vorderste Lamelle 144 c bildet eine konkave, sich in Richtung auf den Boden des Mischbechers 100 öffnende Fläche. Auch die anderen Lamellen können konkav sein. Im Körper 142 ist ein nach oben gasdürchlässiges Nadelventil 146 vorgesehen.

Am oberen Ende des Applikationsbehälters 130 ist ein Kolben oder Stempel 150 mit mehreren Lamellen 152 dargestellt. Der Stempel 150 schliesst gegenüber der Innenwand des Applikationsbehälters 130 zementdicht, aber gasdurchlässig ab. Der Stempel 150 weist ein zentrales Innengewinde 154 auf, in das eine Handhabungsvorrichtung 160 für den Stempel mit einer Stange 162 einschraubbar ist.

Ferner ist in Fig 1 ein Monomerbehälter 170 zur Aufnahme des Monomers 171 dargestellt. Der Monomerbehälter 170 weist eine Nadel oder Spitze 172, einen runden Vorsprung 173 und einen als Standfläche dienenden ebenen Boden 174 auf

Fig. 1 stellt die Ausgangsposition für das erfindungsgemässe Verfahren dar. Das Polymerpulver 176 befindet sich im Applikationsbehälter 130, der distal mit der dünnen Membran 136 verschlossen ist. Die Membran besteht aus einem Material, das durch das verwendete Monomer aufgelöst wird, vorzugsweise aus Knochenzement derselben Zusammensetzung wie der zu mischende Knochenzement, wie PMMA, allerdings ohne Röntgenkontrastmittel und ohne Füller. Der Applikationsbehälter 130 ist am distalen Ende gegen den Mischbecher 100 durch die Abdichteinrichtung 140 und proximal durch den Stempel 150 abgedichtet.

Nach dem Einschrauben der Handhabungsvorrichtung 160 in den Stempel 150 wird zunächst der Monomerbehälter 170 mit dem Vorsprung 173 in die Ausnehmung 104 am Boden des Mischbechers 100 eingepresst und dort in einem Pressitz festgehalten. Dabei durchstösst die zuvor geöffnete Spitze 172 des Monomerbehälters 170 den Stopfen 106, und das Monomer im Monomerbehälter 170 steht mit dem Innenraum des Mischbechers 100 in Verbindung.

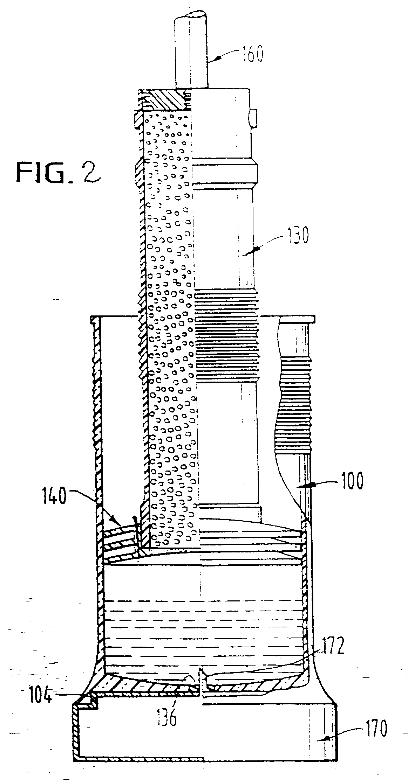

Nun wird der Mischbecher 100 in einer Hand an seiner als Riffelung ausgebildeten Griffläche 108 festgehalten und der mit der Abdichteinrichtung 140 gekoppelte Applikationsbehälter 130 wird mit der anderen Hand gefasst, zurückgezogen und dabei wie eine Injektionsspritze soweit aufgezogen, bis alles Monomer aufgrund des entstehenden Unterdrucks durch die Nadel oder Spritze 172 in den Mischbecher 100 aufgesaugt worden ist. Diese Stellung ist in Fig. 2 dargestellt.

Durch den Kontakt mit dem Monomer und dessen Dampf wird die dünne Membran 136 vollständig aufgelöst. Dies stellt kein Problem dar, da die Membran aus demselben chemischen Material besteht wie der fertige Knochenzement. Durch das Auflösen der Membran 136 wird das Polymerpulver im Applikationsbehälter 130 befreit und gelangt in das Monomer. Das Einbringen des Polymerpulvers in das Monomer weist im Hinblick auf die gleichmässige Verteilung der Polymerkugeln und im Hinblick auf auftretende Lufteinschlüsse Vorteile gegenüber dem Hinzufügen des Monomers zum Polymerpulver auf:

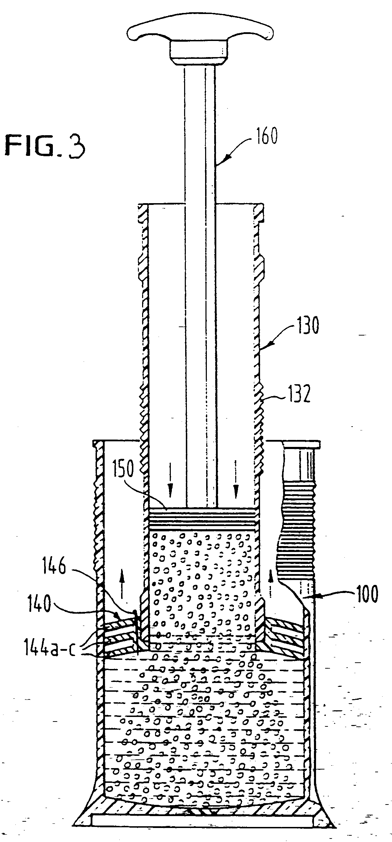

Als nächster Schritt wird der Stempel 150 distal, d.h. nach unten gedrückt, und die Reste der PMMA-Membran 136 werden mit dem Polymerpulver durch den Stempel 150 in das Monomerbad gestossen und aufgelöst. Während sich der Stempel 150 distal bewegt, wird die Abdichteinrichtung 140 mit dem Applikationsbehälter 130 aufgrund der Volumenvergrösserung von Monomer und Polymerpulver im Mischbecher 100 nach proximal, d.h. nach oben geschoben, bis das Polymerpulver vollständig aus dem Applikationsbehälter 130 ausgestossen ist. Die eingeschlossene Luft oder bei der Polymerisation entstehende Gasblasen können dabei zwischen den Lamellen 144 und der Innenwand des Mischbechers 100 und/oder durch das als Nadel-oder Kugelventil ausgebildete Einwegventil 146 austreten. Dieser Schritt ist in Fig. 3 dargestellt, wobei der Monomerbehälter 170 bereits abgezogen ist.

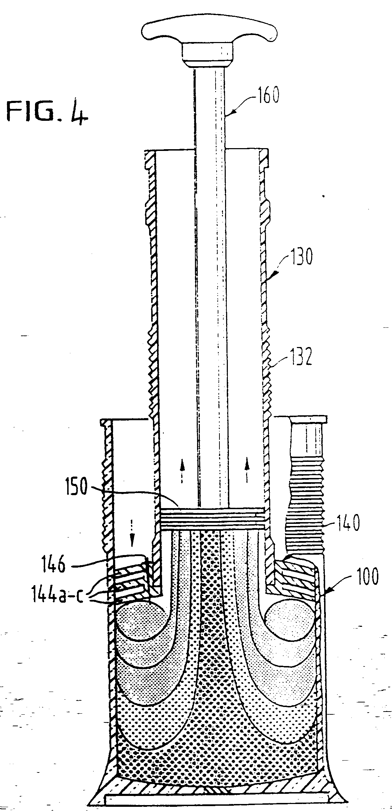

Danach wird der Applikationsbehälter 130 an der als Riffelung ausgebildeten Griffläche 132 gefasst und wieder nach unten gestossen, während der Mischbecher 100 festgehalten oder aufgestellt wird. Da das Rohgemisch aus Polymerpulver und Monomer nicht die Abdichteinrichtung 140 passieren kann, wird durch die Kompression das Rohgemisch aus dem Mischbecher 100 zurück in den Applikationsbehälter 130 extrudiert, wobei das Knochenzement / Rohgemisch den Stempel 150 nach oben drückt. Hierbei kommt es meist zu einer laminaren Schichtung in der Weise, dass die Zementmassen, welche noch am stärksten mit Polymerpulver durchsetzt sind, die radial äussere Randschicht bilden, während die radial inneren Schichten mehr und mehr von Monomer durchsetzt sind. Den Kern des beim Extrudieren entstehenden Kegels bildet das Monomer vom Boden des Mischbechers. Auf diese Weise wird die Lage reinen Polymerpulvers vom flüssigen Monomer durchstossen. Fig 4. zeigt eine derartige Zwischenstellung. Aufgrund der konkaven Form der Lamelle 144c werden Toträume an der Wand des Mischbechers 100 vermieden und das Rohgemisch radial nach innen zur Öffnung des Applikationsbehälters gedrückt, ohne dass Reste an der Wand zurückbleiben. Die laminare Strömung des Gemisches beim Extrudieren durch die Öffnung in den Applikationsbehälter ist in Fig. 4 schematisch dargestellt.

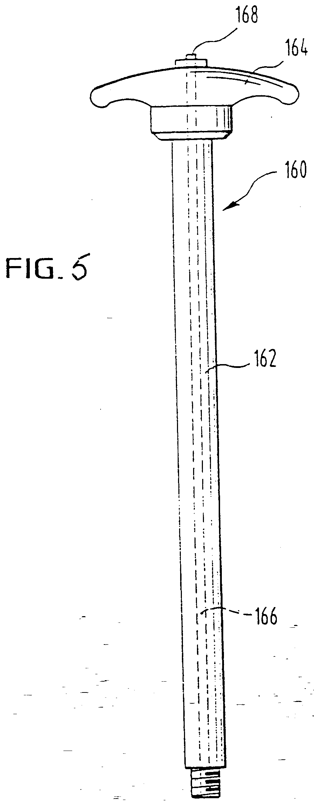

Wenn der Applikationsbehälter 130 bis zum Boden des Mischbechers 100 gedrückt ist, wobei sich die flexible Lamelle 144 c vollständig an die konkave Form des inneren Bodens 112 anpasst, befindet sich das Knochenzementgemisch vollständig in der Kartusche. Danach wird das Gemisch durch Hinunterdrücken des Stempels 150 bei gleichzeitigem Festhalten des Mischbechers 100 wieder zurückgestossen, wobei der Applikationsbehälter 130 vom Gemisch angehoben wird (vgl. Fig 5) und es wiederum zu einer überwiegend laminaren Strömung kommt. Danach wird wieder der Applikationsbehälter 130 von Hand nach unten gedrückt (vgl. Fig 4). Bei einer Wiederholung dieser Verfahrensschritte und abwechselndem Bewegen des Applikationsbehälters 130 und des Stempels 150 nach unten kommt es zur innigen Durchmischung der Schichten. Der Vorgang des Extrudierens kann mehrmals so lange wiederholt werden, bis die Mischung des Knochenzementes vollständig ist. Zum Schluss wird das Knochenzementgemisch endgültig in den Applikationsbehälter 130 extrudiert und der Stempel 150 mit der Handhabungsvorrichtung 160 nach oben herausgenommen. Danach wird durch Lösen des Adapters 138 die Abdichteinrichtung 140 entfernt und mittels eines (nicht dargestellten) Adapters ein Mundstück auf das (in der Zeichnung obere) Vorderende des Applikationsbehälters 130 aufgesetzt, das je nach Anwendung für Pfanne, Femur oder Kniegelenk verschieden sein kann. Nach Aufsetzen des Applikationsbehälters auf eine Knochenzementspritze, z.B. gemäss der EP-A1-170 120, kann der Knochenzement appliziert werden.

Durch die Volumenerhöhung beim Zurückziehen der Abdichteinrichtung entsteht ein Unterdruck über dem Gemisch, der in der Regel ausreicht um eingeschlossene Luftblasen aus dem Gemisch zu entfernen. Diese werden beim Extrudieren nicht mehr in den Knochenzement hineingedrückt, sondern entweichen durch das Ventil 146 in der Abdichteinrichtung 140.

Es kann jedoch auch erwünscht sein, von aussen ein Vakuum anzulegen, um sämtliches Gas aus dem Gemisch zu entfernen.

Fig.5 zeigt eine hierfür geeignete Handhabungsvorrichtung 160 mit einer Stange 162 und einem Handgriff 164. Die Handhabungsvorrichtung weist eine durchgehende innere Durchführung 166 und einen Vakuumstutzen 168 zum Anflanschen einer Vakuumpumpe auf Durch Evakuieren wird das Gas aus dem Mischsystem entfernt.

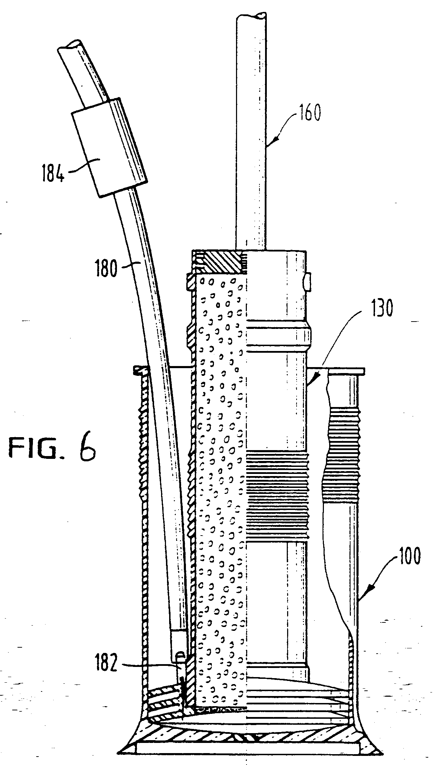

Gemäss Fig 6, die die gleiche Stellung der Vorrichtung wie Fig. 1 zeigt, kann auch eine Vakuumleitung 180 vorgesehen sein, die direkt an das anstelle des Ventils 146 von Fig 3 vorgesehene Ventil 182 angeschlossen ist. Über die Leitung 180 kann eine Evakuierung unter Zwischenschaltung eines Kohlefilters 184 erfolgen. Bei dieser Ausführungsform werden die Luft und andere Gase aus dem Mischbecher 100 abgesaugt, während bei der Ausführungsform nach Fig.5 das Absaugen direkt aus dem Applikationsbehälter 130 erfolgt.

Die Erfindung ist nicht auf die vorstehenden Ausführungsbeispiele beschränkt, die das Mischen und Einfüllen von Knochenzement in einen Applikationsbehälter betreffen, sondern kann allgemein zum Mischen eines aus mindestens zwei Bestandteilen bestehenden Stoffgemisches und Einfüllen des durchmischten Stoffgemisches in einen Behälter verwendet werden. Die vorstehend beschriebenen Ausführungsbeispiele der erfindungsgemäßen Vorrichtung sind daher allgemein zum Mischen eines aus mindestens zwei Bestandteilen bestehenden Stoffgemisches und Einfüllen des Stoffgemisches in einen Behälter anwendbar.

Bei den vorstehend beschriebenen Ausführungsbeispielen wird die zum Mischen und Einfüllen des Gemisches erforderliche Relativbewegung des ersten Behälters, des zweiten Behälters und gegebenenfalls des Stempels durch Betätigung per Hand erzielt. Selbstverständlich kann das erfindungsgemäße Misch- und Füllprinzip, insbesondere auch bei bezüglich des Mischvolumens größeren Systemen, auch durch maschinelle Betätigung der einzelnen Teile realisiert werden. Hierfür können beispielsweise zwei Antriebeinrichtungen vorgesehen sein, von denen eine mit dem zweiten Behälter (Applikationsbehälter) und die andere mit dem Stempel bzw. dessen Handhabungsvorrichtung jeweils lösbar in Eingriff bringbar ist. Durch abwechselndes Ergreifen und Bewegen des zweiten Behälters bzw. des Stempels in Axialrichtung, während gleichzeitig das jeweils andere Bauteil losgelassen wird und frei beweglich ist, kann bei gleichzeitigem Festhaltan des ersten Behälters (Mischbehälters) eine gegenläufige Relativbewegung dar beiden Behälter erzeugt werden, durch die das zu durchmischende Stoffgemisch zwischen den beiden Behältern hin- und herbewegt und durchmischt wird. Es ist auch möglich, jeweils eine in entgegengesetzer Richtung wirkende Antriebseinrichtung für den ersten Behälter (Mischbehälter) und den Stempel bzw. dessen Handhabungsvorrichtung vorzusehen, durch die der erste Behälter bzw. der Stempel abwechselnd in Axialrichtung aufeinander zubewegt werden. Diese Antriebseinrichtungen müssen ebenfalls jeweils lösbar ausgebildet sein, und ferner ist bei dieser Ausführungsform eine Einrichtung zum lösbaren Festhalten des zweiten Behälters (Applikationsbehälters) vorgesehen. Mit einer derartigen Vorrichtung läßt sich ebenfalls die zu dem erläuterten Extrusionsmischen erforderliche Strömungsbewegung des Stoffgemisches zwischen dem ersten und dem zweiten Behälter durch die Öffnung des zweiten Behälters hindurch erzielen.

## Patentansprüche

1. Vorrichtung zum Mischen und Umfüllen eines aus mindestens 2 Bestandteilen bestehenden viskösen Stoffgemisches, mit
a) einem ersten an einem Ende geschlossenen Behälter (100),
b) einem zweiten Behälter (130), dessen Querschnitt kleiner ist als der des ersten Behälters (100), dessen dem ersten Behälter zugewandtes Ende eine Oeffnung aufweist und der im ersten Behälter (100) und relativ zu diesem beweglich ist,
c) einer am äusseren Umfang des zweiten Behälters angeordneten Abdichteinrichtung (140), die bei einer Relativbewegung der beiden Behälter zueinander den Zwischenraum zwischen dem inneren Umfang des ersten Behälters (100) und dem äusseren Umfang des zweiten Behälters (130) für das Stoffgemisch und dessen Bestandteile abdichtet, und
d) einem im zweiten Behälter (130) beweglichen Stempel (150),
gekennzeichnet dadurch, dass der Boden (102) des Behälters (100) einen von einer Nadel (172) durchstossbaren und wiederverschliessbaren Stopfen (106) aufweist.

2. Vorrichtung nach Anspruch 1 dadurch gekennzeichnet, dass das visköse Stoffgemisch Knochenzement ist, der erste Behälter (100) ein Mischbecher mit einem Boden ist und der zweite Behälter (130) zum Applizieren des Stoffgemisches ausgebildet ist.

3. Vorrichtung nach Anspruch 1 oder 2, dadurch gekennzeichnet, dass der im zweiten Behälter (130) bewegliche Stempel (150) den zweiten Behälter für das Stoffgemisch abdichtet.

4. Vorrichtung nach einem der Ansprüche 1 bis 3, dadurch gekennzeichnet, dass der erste Behälter (100) zylindrisch ist und sein Boden (112) innen konkav oder rund ausgebildet ist.

5. Vorrichtung nach einem der Ansprüche 1 bis 4, dadurch gekennzeichnet, dass die Andichteinrichtung (140) mindestens ein Ventil (146; 182) zum Gasdurchlass aufweist.

6. Vorrichtung nach einem der Ansprüche 1 bis 5 , dadurch gekennzeichnet, dass die Abdichteinrichtung (140) einen zylinderförmigen Körper (142) mit mehreren flexiblen Lamellen (144) an seinem Aussenumfang aufweist, die am Innenumfang des ersten Behälters (100) anliegen.

7. Vorrichtung nach Anspruch 6, dadurch gekennzeichnet, dass mindestens die vorderste, dem Boden (112) des ersten Behälters (100) zugewandte Lamelle (144c) konkav ist.

8. Vorrichtung nach einem der Ansprüche 1 bis 7, gekennzeichnet durch einen mit dem ersten Behälter (100) koppelbaren Behälter (170) zur Aufnahme eines Bestandteiles des Stoffgemisches, wobei der dritte Behälter (170) eine Nadel (172) aufweist.

9. Vorrichtung nach einem der Ansprüche 1 bis 8, gekennzeichnet durch eine Membran (136) zum Verschliessen des in dem Behälter (100) einführbaren Endes des zweiten Behälters (130).

10. Vorrichtung nach Anspruch 9, dadurch gekennzeichnet, dass die Membran durch ein an der Bildung des Stoffgemisches beteiligtes Monomer diese auflöst.

11. Vorrichtung nach einem der Ansprüche 1 bis 10, gekennzeichnet durch eine Einrichtung zum Evakuieren des ersten Behälters (100) und / oder des zweiten Behälters (130).

12. Verfahren zum Mischen und Umfüllen eines aus mindestens zwei Bestandteilen bestehenden viskösen Stoffgemisches mittels einer mindestens zwei Behälter aufweisenden Mischvorrichtung, insbesondere mit einer Vorrichtung nach einem der Ansprüche 1 bis 11, dadurch gekennzeichnet, dass das Stoffgemisch mehrfach gegenläufig von einem ersten Behälter durch eine Oeffnung, deren Durchmesser kleiner ist als der Durchmesser mindestens eines der beiden Behälter, in einen zweiten Behälter und wieder zurück gedrückt wird.

## Claims

1. A device for mixing and decanting a viscous mixed substance comprising:
a) a first receptacle (100) closed at one end;
b) a second receptacle (130) with a cross-sectional dimension which is less than the cross-section of said first receptacle (100), wherein the end facing the first receptacle includes an opening, and wherein it is movable in and with respect to said first receptacle (100) ;
c) seal means (140) arranged at the outer circumference of said second receptacle and sealing the space between the inner circumference of said first receptacle (100) and the outer circumference of said second receptacle (130) with regard to the mixed substance and its components during movement of said two receptacles with respect to one another ; and
d) a plunger (150) movable within said second receptacle (130) ; characterised in that the bottom (102) of said receptacle (100) includes a plug (106) being puncturable by a needle (172) and re-sealable.

2. The device of claim 1, characterised in that said viscous mixed substance is bone cement, in that said first receptacle (100) is a mixing beaker, and in that said second receptacle (130) is formed for application of said mixed substance.

3. The device of claim 1 or claim 2, characterised in that said plunger being movable within said second receptacle (130) seals said second receptacle for said mixed substance.

4. The device of claims 1 to 3, characterised in that said first receptacle (200) is cylindrical, and in that the inside of its bottom (112) is provided in concave or round shape.

5. The device of claims 1 to 4, characterised in that the seal means (140) includes at least one valve (146; 182) for allowing gas permeability.

6. The device of claims 1 to 5, characterised in that said seal means (140) includes a cylindrical body (142) with a number of flexible ribs (144) at the outer circumference, said ribs being adjacent to the inner circumference of said first receptacle.

7. The device of claim 6, characterised in that at least the leading rib (144b) facing the bottom (112) of said first receptacle (100) is concave.

8. The device of claims 1 to 7, characterised by a receptacle (170) allowing connection to said first receptacle (100) for receiving a component of said mixed substance, wherein said third receptacle (170) includes a needle (172).

9. The device of claims 1 to 8, characterised by a membrane (136) for closing the end of said second receptacle (130) being insertable into said receptacle (100).

10. The device of claim 9, characterised in that said membrane dissolves through a monomer being part of the formation of said mixed substance.

11. The device of claims 1 to 10, characterised by means for evacuation of said first receptacle (100) and/or said second receptacle (130).

12. A method for mixing and decanting a viscous mixed substance of at least two components by a mixing device including at least two receptacles, in particular with a device of one of the claims 1 to 11, characterised in that said mixed substance being pressed in opposite directions from a first receptacle through an opening with a diameter smaller than the diameter of at least one of the other receptacles into a second receptacle and back.

## Revendications

1. Dispositif permettant de mélanger et de transvaser au moins deux constituants d'un mélange de substances visqueux existant avec
a) un premier récipient (100) fermé à l'une de ses extrémités et caractérisé par le fait que le fond (102) du récipient présente un bouchon (106) qui peut être percé par une aiguille (172) et refermé
b) un second récipient (130) dont la section est plus petite que celle du premier récipient (100) et dont l'extrémité qui est orientée vers le premier récipient présente une ouverture et qui est mobile à l'intérieur du premier récipient (100) et par rapport à celui-ci,
c) un dispositif d'étanchéité (140) disposé sur le pourtour extérieur du second récipient qui réalise l'étanchéité de l'espace intermédiaire existant entre le pourtour intérieur du premier récipient (100) et le pourtour extérieur du second récipient (130) pour le mélange des substances et ses constituants en cas de déplacement relatif des deux récipients l'un par rapport à l'autre, et
d) un tampon mobile (150) se trouvant dans le second récipient (130), caractérisé par le fait que le fond (102) présente un bouchon (106) qui peut être percé par une aiguille (172) et refermé.

2. Dispositif selon la revendication 1, caractérisé par le fait que le mélange de substances visqueux est du cément osseux, le premier récipient (100) étant un bécher qui présente un fond, le second récipient (130) étant configuré de sorte à permettre d'appliquer le mélange de substances.

3. Dispositif selon revendication 1 ou 2, caractérisé par le fait que dans le second récipient (130) le tampon mobile (150) réalise l'étanchéité dans le second récipient (130) pour le mélange des substances.

4. Dispositif selon revendications 1 à 3, caractérisé par le fait que le premier récipient (100) est cylindrique et que son fond (112) est de forme concave à l'intérieur ou ronde.

5. Dispositif selon revendications 1 à 4, caractérisé par le fait que le dispositif d'étanchéité (140) présente au moins une valve (146; 182) pour laisser le gaz s'échapper.

6. Dispositif selon revendications 1 à 5, caractérisé par le fait que le dispositif d'étanchéité (140) présente un corps de forme cylindrique (142) comportant plusieurs lamelles souples (144) sur son pourtour extérieur qui sont appliquées contre le pourtour intérieur du premier récipient (100).

7. Dispositif selon revendication 6, caractérisé par le fait qu'au moins la lamelle qui se trouve en avant et qui est orientée vers le fond (112) du premier récipient (100) est concave.

8. Dispositif selon revendications 1 à 7, caractérisé par la présence d'un récipient (170) qui peut être couplé avec le premier récipient (100) pour recevoir un constituant du mélange de substances, le troisième récipient (170) comportant une aiguille (172).

9. Dispositif selon revendications 1 à 8, caractérisé par une membrane (136) qui permet de fermer l'extrémité du second récipient (130) qui peut être introduite dans le récipient (100).

10. Dispositif selon revendications 9, caractérisé par le fait que la membrane est dissoute par un monomère qui participe à la formation du mélange de substances.

11. Dispositif selon revendications 1 à 10, caractérisé par la présence d'un dispositif d'évacuation du premier récipient (100) et / ou du second récipient (130).

12. Procédé permettant de mélanger et de transvaser au moins deux constituants d'un mélange visqueux de substances existant, au moyen d'un dispositif pour mélange comprenant au moins deux récipients, avec en particulier un dispositif selon revendications 1 à 11, caractérisé par le fait que le mélange de substances est admis à plusieurs reprises et dans des sens contraires à partir d'un premier récipient, par une ouverture dont le diamètre est inférieur à celui d'au moins un des deux récipients, dans un second récipient et inversement.
